(19) Europäisches Patentamt
European Patent Office
Office européen des brevets

(11) **EP 4 180 386 A1**

(12) **EUROPEAN PATENT APPLICATION**

(43) Date of publication:
**17.05.2023 Bulletin 2023/20**

(21) Application number: **21315243.2**

(22) Date of filing: **16.11.2021**

(51) International Patent Classification (IPC):
**C01B 17/04** (2006.01)          **C01B 3/06** (2006.01)
**C01B 3/50** (2006.01)

(52) Cooperative Patent Classification (CPC):
**C01B 17/0495; C01B 3/06; C01B 3/50**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **TotalEnergies OneTech
92400 Courbevoie (FR)**

(72) Inventors:
• **Veryasov, Gleb
  1400 Nivelles (BE)**
• **Vermeiren, Walter
  B-7181 Seneffe (BE)**
• **Nesterenko, Nikolai
  1402 Nivelles (Thines) (BE)**

(74) Representative: **Mellet, Valérie Martine
  Patent 42
  5, rue Dicks
  4081 Esch-sur-Alzette (LU)**

(54) **PROCESS FOR THE CONTINUOUS CONVERSION OF H2S INTO H2 AND SULPHUR**

(57)     The disclosure relates to a process for the continuous conversion of hydrogen sulphide into hydrogen and elemental sulphur using a particular solvent, a particular catalyst and a particular method to extract elemental sulphur from the solvent.

**Fig. 1**

## Description

### Field of the disclosure

The disclosure relates to a process for the continuous conversion of hydrogen sulphide (H2S) into hydrogen ($H_2$) and elemental sulphur ($S_8$) at low temperature.

### Background of the disclosure

Hydrogen sulphide is a major byproduct in the petroleum industry. It is present in large quantities (up to 50%) in gas-condensate fields of natural gas. It is the major product of many minerals and organic substance decomposition. At the same time, hydrogen sulphide is a strong toxic substance causing the poisoning of living organisms. Yet hydrogen sulphide can be a feedstock for producing valuable chemical product-hydrogen.

Hydrogen sulphide is directly thermally decomposed into hydrogen and sulphur according to the reaction:

$$H_2S \rightarrow H_2 + S$$

It is a strongly endothermic process and can have a visible rate only at high temperatures. A method for thermal decomposition of hydrogen sulphide for obtaining hydrogen and sulphur is known. The method comprises passing hydrogen sulphide containing gas through a reaction zone at a temperature in the range from 850 to 1600°C where decomposition of $H_2S$ results in obtaining hydrogen and sulphur, and consequent cooling of said gas to a temperature of 110-150°C for condensing obtained sulphur.

In particular, US 2005/0191237 discloses a process for obtaining hydrogen and sulphur from hydrogen sulphide in which $H_2S$ is firstly dissociated in via a thermal dissociation step followed by a separating step to separate the hydrogen hence formed. Temperature ranging from 1500 to 2000°C are used in the first dissociation step.

The known method has a few disadvantages: high temperature is required for achieving a high degree of hydrogen sulphide decomposition; high energy is consumed for performing the reaction and compensating possible heat loss; there is a potential decrease in decomposition level due to reverse interaction of hydrogen and sulphur on cooling; the method is unsuitable for treating gases containing hydrocarbons and other admixtures that can be pyrolyzed at high temperature; there is low effectiveness of the process when the concentration of hydrogen sulphide in the hydrogen sulphide containing feed decreases; there are requirements to use special expensive materials of high thermal resistance to form a high-temperature reaction zone.

Industrially the conversion of $H_2S$ is done in the Claus unit where $H_2S$ is firstly converted into $SO_2$ in a burner followed by the reaction of this $SO_2$ obtained with $H_2S$ to produce $S_2$. The reactions are summarized below:

$$2\,H_2S + 3\,O_2 \rightarrow 2\,SO_2 + 2\,H_2O$$

$$2\,H_2S + SO_2 \rightarrow 3/2\,S_2 + 2\,H_2O$$

The Claus process is costly in terms of energy, relatively high temperatures are required to perform the process steps. There is a need for finding a process requiring less energy.

Other processes have been proposed at a lower temperature. In particular, US 2016/0333277 discloses a process for removing the sulphur compounds via a first step of adsorption on transition metal sulphides being an adsorbent. In a second step, the adsorbent is regenerated. A high temperature is required at the adsorption step, at least 200°C. Due to the adsorption and desorption steps, the process is discontinuous.

Similarly, RU 2216506 describes the conversion of $H_2S$ over a solid material. Such material is capable of adsorbing hydrogen sulphide and converting it into $H_2$ and sulphur. Frequent regeneration of the material is performed by decomposition of adsorbed $H_2S$ at a temperature not higher than 350°C. On the other hand, RU 2239594 describes the decomposition of $H_2S$ over various materials at low temperature. Once $H_2S$ is adsorbed, the materials are regenerated at a temperature above the melting point of Sulphur *i.e.* below 110-120°C.

Recently, a new process running at ambient temperature has been proposed. In particular, in the study entitled "Low-temperature catalytic decomposition of hydrogen sulphide into hydrogen and diatomic gaseous sulphur" (Startsev A. N., et al., Top. Catal., 2013, 56 (11), 969-980), the possibility to convert $H_2S$ over a catalyst immersed in a 5% aqueous solution of monoethanolamine (MEA) was disclosed. This solvent is capable of dissolving the sulphur generated during the conversion of $H_2S$ over the catalyst. With the use of such solvent, conversion of 100% can be achieved. But the recovery of $S_8$ produced during the reaction and dissolved in the solvent appears to be difficult.

WO 2017/026914 describes the decomposition of $H_2S$ at low temperature (0-35°C) via the contact of $H_2S$ with a catalyst (stainless steel) and sulphur adsorbent (ethanolamine) loaded into sequential installed modules. The addition of 6-12 modules allows converting 75% of $H_2S$. However, the process described still require a regeneration step performed with nitrogen for sulphur desorption as well as a clean-up step for trapping unreacted H2S with an amine. Such a need for regeneration renders the process uneconomical.

Similarly, the study entitled "Low-temperature chemisorption-enhanced catalytic decomposition of hydrogen sulphide: Thermodynamic analysis and process concept" (Zagoruiko A., Catalysis Today, 2019, 329, 171-176) discloses a process based on an unsteady-state approach with two reaction stages: (a) the chemisorption of hydrogen sulphide with sulphides of transient metals (Fe, Co, Ni, etc.) together with the emission of hydrogen and formation of metal disulphides at ambient temperature and (b) the backward decomposition of disulphides to sulphur and initial sulphides at a temperature ranging from 200-300°C. The need for a regeneration step is again a clear drawback of this process.

US 2007/0292337 describes the decomposition of $H_2S$ and mercaptans over a catalyst at a temperature lower than 200°C with the help of the immersion of such catalyst in a liquid phase. The liquids used include benzene, liquid diesel oil, diethanolamine in water, ammonia and water. Different catalysts such as graphite carbon material, $MoS_2$, sulphidated cobalt or CoMoS deposited on alumina were used. The decomposition of $H_2S$ leads to the release of $H_2$ gaseous and sulphur is formed on the catalyst surface. The catalyst is placed in a liquid medium capable of dissolving sulphur forming on the surface of the solid material. However, this process is silent on how to recover the sulphur from the solvent.

There is therefore still a need for an improved process for continuous conversion of $H_2S$ into hydrogen together with easy recovery of the sulphur produced. There is also a need for a process that encompasses the recovery of sulphur produced.

It has been found that the catalytic conversion.of $H_2S$ into hydrogen and sulphur can be.performed at ambient temperature and that the sulphur produced can be easily recovered when a particular solvent treatment is used.

## Summary of the disclosure

The present disclosure, which is described below and defined by the claims, offers a novel process for the conversion of $H_2S$ into hydrogen and elemental sulphur $S_8$ together with an improved process for the recovery of the elemental sulphur $S_8$.

In a first aspect, the disclosure concerns a process for the continuous conversion of hydrogen sulphide into hydrogen and elemental sulphur, the process comprising the following steps:

a) providing a first stream comprising at least 1 wt.% of hydrogen sulphide based on the total weight of said first stream;
b) providing a second stream comprising a solvent said solvent comprising one or more organic compounds having a C-N bond wherein the content of the one or more organic compounds having a C-N bond in the second stream is ranging from 1 to 90 wt.% based on the total weight of said second stream;
c) providing a catalyst composition;
d) putting in contact said first stream with said second stream and with said catalyst composition under reaction conditions comprising a temperature ranging between 0°C and 100°C and a pressure ranging between 0.01 MPa and 10.0 MPa, to at least partially convert hydrogen sulphide into a first effluent comprising the solvent, elemental sulphur, hydrogen, and unconverted hydrogen sulphide;
e) recovering said first effluent and performing a separation on said first effluent to provide at least a third stream comprising the solvent and elemental sulphur;

said process is **remarkable in that** it further comprises the step (f) of removing the solvent from the third stream to recover a second effluent comprising elemental sulphur and a third effluent comprising the solvent, wherein said step (f) is performed by a distillation selected from a distillation or steam distillation; or is performed by an extraction selected from an extraction over an adsorbent or an extraction over carbon dioxide or an extraction over sulphur dioxide.

Surprisingly, it has been discovered that the elemental sulphur produced can be recovered via distillation, $CO_2$ extraction or steam distillation or extraction over an adsorbent. The choice of the method of recovery of elemental sulphur in the solvent is versatile and depends on the solvent used.

A process for the continuous conversion of hydrogen sulphide and elemental sulphur has, therefore, been developed. The solvent comprising one or more organic compounds having a C-N bond which is used to dissolve the hydrogen sulphide and to enhance its decomposition can be recovered thanks to the process of the disclosure, which may allow for further recycling of such solvent. Using different techniques such as distillation or extraction has allowed recovery of not only the solvent but also the elemental sulphur that is produced during the decomposition of hydrogen sulphide over a catalyst. The increase of the recovery rate of elemental sulphur has been demonstrated.

For example, the first stream further comprises at least 1 wt.% of carbon oxides based on the total weight of said first stream, said carbon oxides being selected from carbon monoxide, carbon dioxide and any mixture thereof, and the first effluent of step d) further comprises carbon oxides.

For example, the elemental sulphur remaining in the solvent after said step (f) is below 1.0 wt.% based on the total weight of the third effluent, preferably below 0.1 wt.%, even more preferably below 0.01 wt.%.

For example, the second effluent comprises at least 95 wt.% of elemental sulphur based on the total weight of the second effluent, preferably at least 97 wt.%, more preferably at least 99 wt.% or the second effluent only comprises elemental sulphur.

Advantageously, the second effluent is further treated by distillation and/or by sublimation.

**In a first implementation,** said step (f) of removing the solvent from the third stream is performed by distillation.

- For example, the content of the one or more organic compounds having a C-N bond in the second stream is ranging from 1 to 50 wt.% based on the total weight of said second stream; the second stream further comprises one or more hydrocarbons at a content ranging from 1 to 50 wt.% based on the total weight of said second stream and the step (f) of removing the solvent from the third stream is performed by distillation. With preference, said distillation is carried out at a temperature ranging between above 150°C and below 450°C and/or the second stream further comprises from 5 to 45 wt.% of water based on the total weight of said second stream.
- With preference, said distillation is carried out at a temperature of ranging between 155°C and 445°C, more preferably between 160°C and 440°C, even more preferably between 170°C and 430°C, most preferably between 180°C and 420°C.
- With preference, said distillation is carried out at a pressure ranging between 0.0001 MPa and a pressure of 10 MPa, more preferably between 0.001 MPa and 1 MPa.
- Advantageously, the solvent of the second stream further comprises carbon disulphide, preferably at a concentration ranging from 1 to 90 wt.% based on the total weight of said second stream, more preferably from 5 to 80 wt.%, even more preferably from 10 wt.% to 70 wt.%, most preferably from 15 wt.% to 60 wt.%, even most preferably from 20 wt.% to 50 wt.%.

**In a second implementation,** said step (f) of removing the solvent from the third stream is performed by steam distillation. For example, said steam distillation comprises the following sub-steps: (f1) stripping the third stream with steam to recover the third effluent and (f2) recovering the second effluent by decantation and/or filtration.

- For example, the second stream further comprises one or more hydrocarbons at a content ranging from 1 to 50 wt.% based on the total weight of said second stream and the step (f) of removing the solvent from the third stream is performed by steam distillation; wherein the steam distillation comprises the following sub-steps: (f1) stripping the third stream with steam to recover the third effluent and (f2) recovering the second effluent by decantation and/or filtration
- Optionally, a sixth effluent comprising hydrogen sulphide is recovered from step (f1). When the sixth effluent is recovered, the process advantageously comprises the step of directing said sixth effluent into the first stream provided in step (a) and/or used in step (d).
- For example, the sub-step (f1) is carried out at a temperature of at least 105°C, preferably at least 110°C, more preferably at least 115°C, more preferably at least 120°C or at least 130°C.
- Advantageously, the solvent of the second stream further comprises carbon disulphide, preferably at a concentration ranging from 1 to 90 wt.% based on the total weight of said second stream, more preferably from 5 to 80 wt.%, even more preferably from 10 wt.% to 70 wt.%, most preferably from 15 wt.% to 60 wt.%, even most preferably from 20 wt.% to 50 wt.%.

**In a third implementation,** said step (f) of removing the solvent from the third stream is performed by extraction over carbon dioxide or sulphur dioxide. For example, said extraction over carbon dioxide or sulphur dioxide comprises the following sub-steps: (f1) addition of carbon dioxide or sulphur dioxide on the third stream to form a saturated solution, (f2) heating the saturated solution at a temperature of at least 100°C and (f3) recovering the second effluent, the third effluent, and optionally a carbon dioxide stream or a sulphur dioxide stream; with preference:

- the second stream further comprises from 5 to 50 wt.% of water based on the total weight of said second stream; and/or
- the sub-step (f2) is performed at a temperature of at least 110°C, more preferably at least 120°C or at least 130°C; and/or
- the sub-step (f3) is performed by decantation or filtration.

Advantageously, the solvent of the second stream is devoid of carbon disulphide.

**In a fourth implementation,** said step (f) of removing the solvent from the third stream is performed by extraction over an adsorbent. For example, said extraction over an adsorbent comprises the following sub-steps: (f1) recovering of the third effluent by adsorption on an adsorbent of the third stream, and (f2) recovering of the second effluent by heating said adsorbent at a temperature of at least 100°C.

- With preference, said adsorbent is one or more selected from copper oxide, zinc oxide, amorphous aluminum, zeolite, hydrated alumina, crystalline boehmite, pseudo-boehmite, and gibbsite.
- For example, said adsorbent is blended with a binder, such as alumina, silica, zirconia or any mixtures thereof.
- For example, said adsorbent is blended with a diluent, such as a metal or a transition metal.
- Advantageously, the sub-step (f2) is performed at a temperature of at least 110°C, preferably at least 120°C, more preferably at least 130°C, even more preferably at least 140°C.
- Advantageously, the solvent of the second stream is devoid of carbon disulphide.

Whatever the implementation employed, one or more of the following features can be advantageously defined for describing the first stream provided at step (a):

- The first stream is a gaseous stream.
- The first stream comprising at least 30 wt.% of hydrogen sulphide based on the total weight of said first stream, preferably at least 50 wt.%, more preferably at least 75 wt.%, even more preferably at least 90 wt.%, most preferably at least 95 wt.% or at least 99 wt.%, or the first stream comprises only hydrogen sulphide.
- The first stream further comprises at least 1 wt.% of carbon oxides based on the total weight of said first stream, said carbon oxides being selected from carbon monoxide and/or carbon dioxide, more preferably at least 5 wt.%, even more preferably at least 15 wt.%; or said first stream comprises between 1 wt.% and 50 wt.% of carbon oxides based on the total weight of said first stream, more preferably between 5 wt.% and 45 wt.%, even more preferably between 15 wt.% and 35 wt.%, said carbon oxides being selected from carbon monoxide and/or carbon dioxide.

Whatever the implementation employed, one or more of the following features can be advantageously defined for describing the second stream provided at step (b):

- The second stream is a liquid stream.
- The second stream further comprises water at a content ranging from 1 to 50 wt.% based on the total weight of the second stream, preferably from 5 to 45 wt.%, more preferably from 10 to 40 wt.%.
- The one or more organic compounds having a C-N bond are capable of solubilizing hydrogen sulphide.
- The one or more organic compounds having a C-N bond are one or more selected from diethanolamine, monoethanolamine, methyldiethanolamine, diisopropanolamine, digycolamine and any mixture thereof; preferably, the one or more organic compounds having a C-N bond is monoethanolamine.
- The content of the one or more organic compounds having a C-N bond in the second stream is ranging from 1 to 50 wt.% based on the total weight of said second stream; preferably ranging from 1 to 90 wt.%.
- The content of the one or more organic compounds having a C-N bond in the second stream is ranging from 1 to 90 wt.% based on the total weight of said second stream, preferably from 5 to 80 wt.%, more preferably from 10 wt.% to 70 wt.%, even more preferably from 15 wt.% to 60 wt.%, most preferably from 20 wt.% to 50 wt.%, even most preferably from 25 wt.% to 45 wt.%.
- The second stream further comprises carbon disulfide.
- The second stream further comprises carbon disulfide at at content ranging from 1 wt.% to 90 wt.% based on the total weight of said second stream, preferably from 5 wt.% to 80 wt.%; more preferably from 10 wt.% to 70 wt.%, even more preferably from 15 wt.% to 60 wt.%.
- The second stream further comprises one or more hydrocarbons.
- The second stream further comprises one or more hydrocarbons and the content of the one or more hydrocarbons in the second stream is ranging from 1 to 50 wt.% based on the total weight of said second stream, preferably from 5 to 45 wt.%, more preferably from 10 to 40 wt.%.
- The second stream further comprises one or more hydrocarbons selected from 1,2-dichloroethane, carbon tetrachloride, nitrobenzene, chloroform, cyclohexane, aniline, pyridine, *m*-xylene, *p*-xylene, *o*-xylene, toluene, benzene, chlorobenzene, 1,2-dibromoethane, dichloromethane, diphenyl ether, biphenyl, any C5 to C10 alkanes, any C5-C10 alkenes, and any mixture thereof; with preference, the one or more hydrocarbons are or comprise toluene.

In a preferred embodiment, the one or more organic compounds having a C-N bond, the water, the one or more hydrocarbons and the carbon disulphide if any are mixed before step (d), to form a uniform phase. For example, the one or more organic compounds having a C-N bond, the water, the one or more hydrocarbons, the carbon disulphide if any and the third effluent are mixed before step (d), to form a uniform phase.

Whatever the implementation employed, one or more of the following features advantageously define the catalyst composition provided at step (c):

- The catalyst composition provided at step (c) comprises a catalyst being one or more selected from alkali metal sulphide, alkali earth metal sulphide, transition metal sulphide, alkali metal hydrosulfite, alkali earth metal hydrosulfite, transition metal hydrosulfite, alkali metal polysulphide, alkali earth metal polysulphide, transition metal polysulphide.
- The catalyst composition provided at step (c) comprises a catalyst being one or more transition metal sulphides. For example, the catalyst of the catalyst composition has a spherical shape.
- The catalyst composition provided at step (c) is provided in the form of a catalytic bed.

Whatever the implementation employed, one or more of the following features advantageously define the reaction conditions of step (d):

- A temperature ranging between 0°C and 100°C, preferably between 5°C and 80°C, more preferably between 10°C to 50°C.
- A pressure ranging between 0.01 MPa and 10.0 MPa, preferably between 0.1 MPa and 5.0 MPa or between 0.1 MPa and 1.0 MPa.
- A gaseous hourly space velocity of the first stream ranges between 0.01 $s^{-1}$ and 500 $s^{-1}$, preferably between 0.1 $s^{-1}$ and 300 $s^{-1}$; more preferably between 1 $s^{-1}$ and 250 $s^{-1}$.
- A weight hourly space velocity of the second stream ranging between 0.01 $s^{-1}$ and 500 $s^{-1}$, preferably between 0.1 $s^{-1}$ and 300 $s^{-1}$; more preferably between 1 $s^{-1}$ and 250 $s^{-1}$.

Whatever the implementation employed, the step. (e) of recovering said first effluent and performing a separation on said first effluent advantageously further provides at least a fourth stream comprising hydrogen, unconverted hydrogen sulphide if any and carbon oxides when carbon oxides are present in said first stream. In this case, one or more of the following features further advantageously define the process:

- Said process further comprises a step (h) of performing a separation on said fourth stream to provide at least a fourth effluent comprising the unconverted hydrogen sulphide if any.

  ◦ For example, said process further comprises the step of directing said fourth effluent into the first stream provided in step (a) and/or used in step (d).
  ◦ For example, the weight ratio of the flow rate of the fourth effluent into the first stream provided in step (a) is less than 0.50, preferably less than 0.10.

- Said process further comprises a step (h) of performing a separation on said fourth stream (9) to provide at least a fifth effluent comprising hydrogen and carbon oxides when carbon oxides are present in said first stream.

  ◦ Optionally, said process further comprises a step (k) of providing a reverse water gas shift catalyst and a step (I) of contacting said fifth effluent with said reverse water gas shift catalyst to form a CO-enriched effluent comprising at least hydrogen, carbon dioxide and carbon monoxide.
  ◦ For example, said process further comprises a step (i) of providing a methanol synthesis catalyst and step (j) of contacting said fifth effluent and/or said CO-enriched effluent if formed with said methanol synthesis catalyst to produce a sixth effluent comprising at least methanol.

Whenever the process comprises steps (i) and (j), the sixth effluent advantageously comprises methanol, unconverted hydrogen, unconverted carbon dioxide, unconverted carbon monoxide and/or water. Said sixth effluent is advantageously separated, preferably via condensation, into a liquid effluent comprising methanol and/or water and a gaseous stream comprising hydrogen, carbon dioxide and/or carbon monoxide. Advantageously, a distillation step can be further performed onto said liquid effluent.

Whatever the implementation employed, one or more of the following features advantageously define the process of the present disclosure:

- Said step (d) is performed in a trickle bed mode.
- When the catalyst composition provided at step (c) is provided under the form of a catalytic bed, the second stream and the first stream are injected downstream said catalyst bed, said step (d) is performed in a trickle bed mode and said step (e) is performed on the top of the catalyst bed.
- Said step (d) and said step (e) are performed simultaneously.
- The weight ratio of the flow rate of the second stream to the first stream ranges between 100/1 to 1/100, preferably between 90/1 to 10/100, more preferably between 80/1 to 20/100.
- Said process further comprises a step (g) of recycling said third effluent at step (d). For example, said third effluent is mixed with the second stream before step (d). For example, the weight ratio between the second stream and the third effluent is ranging between 0.01 to 1.00, preferably between 0.05 and 0.95, more preferably between 0.10 and 0.90, even more preferably between 0.15 and 0.85.

For example, the first effluent comprises at most 5 wt.% of unconverted hydrogen sulphide based on the total weight of said first effluent, preferably at most 1 wt.%, more preferably at most 0.1 wt.% or the first effluent is free of hydrogen sulphide.

For example, the fifth effluent comprises at least 90 wt.% of hydrogen based on the total weight of said fifth effluent or at least 95 wt.%, preferably at least 97 wt.%, more preferably at least 99 wt.% or the fifth effluent only comprises hydrogen.

In a second aspect, the disclosure concerns an installation to perform the process according to the first aspect said installation comprising

- a reaction unit,
- a separation unit and
- a sulphur recovering unit,

wherein the reaction unit, the separation unit and the sulphur recovering unit are fluidically connected in series, the separation unit being downstream of the reaction unit and upstream of the sulphur recovering unit, said installation is remarkable in that said sulphur recovering unit comprises at least one distillation apparatus, or at least one stripping apparatus, or at least one extraction apparatus, or least one adsorbent.

With preference, said installation further comprises a line directed between the sulphur recovering unit and the reaction unit to direct the third effluent from the sulphur recovering unit to the reaction unit.

With preference, said installation further comprises a hydrogen recovering unit, said hydrogen recovering unit being fluidically connected in series to the separation unit and being downstream of said separation unit. Advantageously, said installation further comprises a line directed between the hydrogen recovering unit and the reaction unit to direct the fourth effluent from the hydrogen recovering unit to the reaction unit.

When said installation comprises a hydrogen recovering unit, said installation advantageously further comprises a methanol synthesis unit, said methanol synthesis unit being fluidically connected in series to the hydrogen recovering unit and being downstream of said hydrogen recovering unit. Optionally, said installation further comprises a reverse water gas shift unit, said reverse water gas shift unit being fluidically connected in series to the hydrogen recovering unit and being downstream of said hydrogen recovering unit and upstream of said methanol synthesis unit. Advantageously yet, a methanol separation unit is fluidically connected in series to the methanol synthesis unit and is downstream of said methanol synthesis unit. For example, the installation comprises a line exiting the methanol separation unit and directed to at least one or preferably both the methanol synthesis unit and the reverser water gas shift unit. Optionally, said methanol synthesis unit and/or said reverse water gas shift unit are also fed with a first line of hydrogen and/or a second line of carbon dioxide and/or a third line of carbon monoxide.

Advantageously, the reaction unit comprises a fixed bed reactor. One or more of the following features advantageously defines the fixed bed reactor of the reaction unit:

- The fixed bed reactor has a catalyst bed comprising stainless steel, wherein said stainless steel is preferably one or more selected from austenitic stainless steel, ferritic stainless steel, duplex stainless steel, martensitic, stainless steel, precipitation hardening stainless steel or any mixture thereof. For example, stainless, steel is packed stainless steel, preferably having a packed surface of at least 50 $m^2/m^3$. For example, the stainless steel is in the form of Raschig rings.
- The fixed bed reactor is a trickle bed reactor, wherein the first stream and the second stream are introduced co-currently or counter-currently; preferably co-currently. For example, the fixed bed reactor or the trickle bed reactor comprises a flow repartition unit.
- The fixed bed reactor is a trickle bed reactor and said step (d) and said step (e) are performed simultaneously.

**Definitions**

For the disclosure, the following definitions are given:
The terms "alkane" or "alkanes" as used herein describe acyclic branched or unbranched hydrocarbons having the general formula $C_nH_{2n+2}$, and therefore consisting entirely of hydrogen atoms and saturated carbon atoms; see e.g.

IUPAC. Compendium of Chemical Terminology, 2nd ed. (1997). The term "alkanes" accordingly describes unbranched alkanes ("normal-paraffins" or "n-paraffins" or "n-alkanes" or "paraffins") and branched alkanes ("iso-paraffins" or "iso-alkanes") but excludes naphthenes (cycloalkanes). They are sometimes referred to by the symbol "HC-".

The term "hydrocarbon" refers to the alkanes (saturated hydrocarbons), cycloalkanes, aromatics and the alkenes (unsaturated hydrocarbons) and dienes together.

As used herein, the terms "C# alcohols", "C# alkenes", or "C# hydrocarbons", wherein "#" is a positive integer, is meant to describe respectively all alcohols, alkenes or hydrocarbons having # carbon atoms. Moreover, the term "C#+ alcohols", "C#+ alkenes", or "C#+ hydrocarbons", is meant to describe all alcohol molecules, alkene molecules or hydrocarbons molecules having # or more carbon atoms. Accordingly, the expression "C5+ alcohols" is meant to describe a mixture of alcohols having 5 or more carbon atoms.

Weight hourly space velocity (WHSV) is defined as the hourly weight flow per unit weight of catalyst and liquid hourly space velocity (LHSV) is defined as the hourly volume of flow per unit of volume of catalyst.

The terms "comprising", "comprises" and "comprised of" as used herein are synonymous with "including", "includes" or "containing", "contains", and are inclusive or open-ended and do not exclude additional, non-recited members, elements or method steps. The terms "comprising", "comprises" and "comprised of" also include the term "consisting of".

The recitation of numerical ranges by endpoints includes all integer numbers and, where appropriate, fractions subsumed within that range (e.g. 1 to 5 can include 1, 2, 3, 4, 5 when referring to, for example, a number of elements, and can also include 1.5, 2, 2.75 and 3.80, when referring to, for example, measurements). The recitation of endpoints also includes the recited endpoint values themselves (e.g. from 1.0 to 5.0 includes both 1.0 and 5.0). Any numerical range recited herein is intended to include all sub-ranges subsumed therein.

The term "conversion" means the mole fraction (i.e., percent) of a reactant converted to a product or products. The term "selectivity" refers to the percent of converted reactant that went to a specified product.

The terms "wt.%", "vol.%", or "mol.%" refers to a weight, volume, or molar percentage of a component, respectively, based on the total weight, the total volume of material, or total moles, that includes the component. In a non-limiting example, 10 grams of components in 100 grams of the material is 10 wt. % of components.

The term "transition metal" refers to an element whose atom has a partially filled d sub-shell, or which can give rise to cations with an incomplete dsub-shell (IUPAC definition). According to this definition, the transition metals are Sc, Ti, V, Cr, Mn, Fe, Co, Ni, Cu, Zn, Y, Zr, Nb, Mo, Tc, Ru, Rh, Pd, Ag, Cd, La, Hf, Ta, W, Re, Os, Ir, Pt, Au, Hg, Ac, Rf, Db, Sg, Bh, Hs, Mt, Ds, Rg, and Cn. The metals Ga, In, Sn, Tl, Pb and Bi are considered as "post-transition" metal.

A trickle-bed reactor (TBR) is a chemical reactor that uses the downward movement of a liquid and the downward (co-current) or upward (counter-current) movement of gas over a packed bed of (catalyst) particles. This reactor allows performing catalytic reactions where gas and liquid are present in the reactor.

A batch reactor consists of a tank with an agitator with an optionally integrated heating or cooling system.

## Description of the figures

- Figure 1 presents a configuration to convert a hydrogen sulphide stream in accordance with the disclosure.
- Figure 2 presents a configuration to convert a hydrogen sulphide stream comprising also carbon oxides in accordance with the disclosure.
- Figure 3 presents a possible trickle bed reactor configured in a co-current configuration that can be used in the process.
- Figure 4 presents an SEM picture of sulphur deposits isolated from the reactor.
- Figure 5 presents an EDX profile of sulphur deposits isolated from the reactor.
- Fugure 6 presents a Raman spectrum of sulphur deposits isolated from the reactor.

## Detailed description of the disclosure

The disclosure concerns a process for the continuous conversion of hydrogen sulphide ($H_2S$) into hydrogen ($H_2$) and elemental sulphur ($S_8$), the process comprising the following steps:

a) providing a first stream 1 comprising at least 1 wt.% of hydrogen sulphide ($H_2S$) based on the total weight of said first stream 1;
b) providing a second stream 3 comprising a solvent said solvent comprising one or more organic compounds having a C-N bond wherein the content of the one or more organic compounds having a C-N bond in the second stream 3 is ranging from 1 to 90 wt.% based on the total weight of said second stream 3;

c) providing a catalyst composition;

d) putting in contact said first stream 1 with said second stream 3 and with said catalyst composition under reaction conditions comprising a temperature ranging between 0°C and 100°C and a pressure ranging between 0.01 MPa and 10.0 MPa, to at least partially convert hydrogen sulphide ($H_2S$) into a first effluent 5 comprising the solvent, elemental sulphur ($S_8$), hydrogen (H2) and unconverted hydrogen sulphide ($H_2S$) if any;

e) recovering said first effluent 5 and separating from said first effluent 5 a third stream 7 comprising the solvent and elemental sulphur ($S_8$) and a fourth stream 9 comprising hydrogen ($H_2$) and unconverted hydrogen sulphide ($H_2S$) if any;

said process is **remarkable in that** it further comprises the step (f) of removing the solvent from the third stream 7 to recover a second effluent 11 comprising elemental sulphur ($S_8$) and a third effluent 13 comprising the solvent, wherein said step (f) is performed by a distillation selected from a distillation or steam distillation: or is performed by an extraction selected from an extraction over an adsorbent or an extraction over carbon dioxide or an extraction over sulphur dioxide.

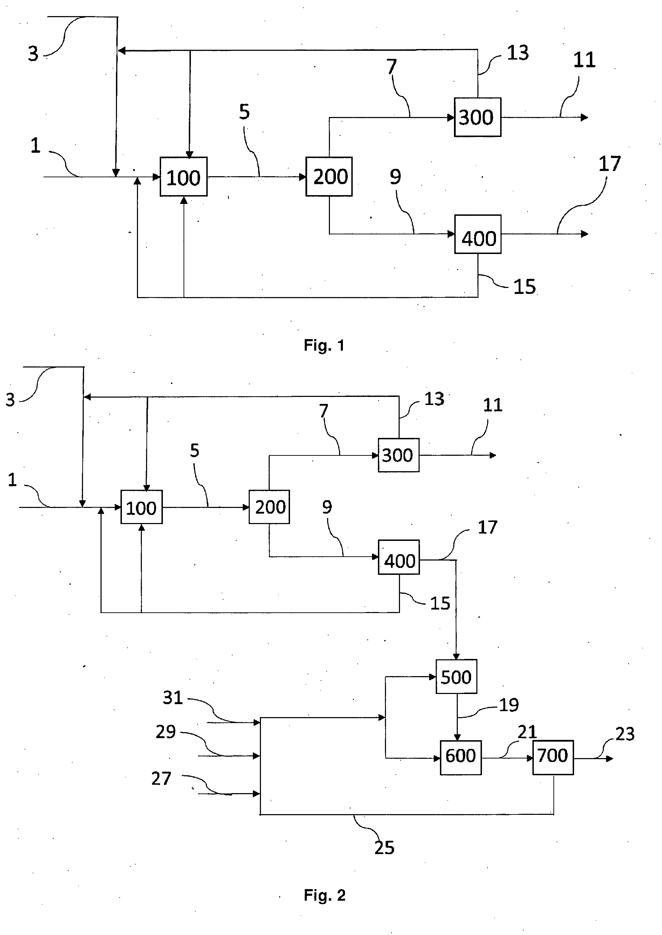

**Figure 1** represents an installation suitable to perform the above process. A reaction unit 100, comprising the catalyst composition provided in step (c) allows the decomposition of the first stream 1 comprising at least 1 wt.% of hydrogen sulphide based on the total weight of said first stream 1 in a solvent provided in the second stream 3 and comprising one or more organic compounds having a C-N bond. The first stream 1 can optionally comprise at least 1 wt.% of carbon oxides based on the total weight of said first stream 1, for example between 1 wt.% and 50 wt.% of carbon oxides based on the total weight of said first stream 1, said carbon oxides being selected from carbon monoxide and/or carbon dioxide. The solvent provided in the second stream 3 allows the dissolution of the first stream 1 and enhances the decomposition of hydrogen sulfide into hydrogen and elemental sulphur. The first effluent 5 exiting the reaction unit 100 is recovered and comprises the solvent, elemental sulphur, hydrogen, unconverted hydrogen sulfide if any and carbon oxides when carbon oxides are present in the first stream 1. The first effluent 5 is conveyed into a separation unit 200 which allows a gas-liquid separation from the first effluent 5 into at least a third stream 7.

To recover the elemental sulphur, the third stream 7, comprising the solvent and the elemental sulphur, is directed into a sulphur recovering unit 300. In there, according to one of the four implementations described in the present disclosure (distillation, steam distillation, $CO_2$ or $SO_2$ extraction, extraction over an adsorbent), it is possible to recover a second effluent 11 that comprises elemental sulphur and a third effluent 13 that comprises the solvent. The third effluent 13 is advantageously recycled through a line in the reaction unit 100 or in the second stream 3.

To recover hydrogen, the first effluent 5 can also be separated into the separation unit 200 into a fourth stream 9, which comprises hydrogen and unconverted hydrogen sulfide if any, is directed into a hydrogen recovering unit 400. For example, the fourth stream 9 is a gaseous stream. In there, it is possible to separate a fourth effluent 15 comprising the unconverted hydrogen sulfide and a fifth effluent 17 comprising at least hydrogen. Such separation could be performed by adsorption, absorption, or distillation. The fifth effluent 17 can also comprise carbon oxides when said carbon oxides are present in the first stream 1. The fourth effluent 15 is advantageously recycled through a line in the reaction unit 100 or in the first stream 1.

The installation represented in figure 1 is useful when the first stream 1 comprises few carbon oxides such as $CO_2$ and/or CO, for example in an amount below 1.0 wt.% based on the total weight of said first stream 1.

In another embodiment, it is possible to take advantage of the presence of carbon oxides in the first stream 1 to generate methanol. This is implemented by steps (i) and (j) of the process. This is useful when the first stream 1 comprises a bigger amount of carbon oxides such as $CO_2$ and/or CO, for example in an amount of at least 1.0 wt.% based on the total weight of said first stream 1. The installation allowing the generation of methanol is represented in figure 2, which shows the installation of figure 1 at which a methanol synthesis unit 600 has been added downstream of the hydrogen recovery unit 400. The methanol synthesis unit 600, which comprises a methanol synthesis catalyst, allows converting the fifth effluent 17 comprising hydrogen and carbon oxides into a sixth effluent 21 comprising at least methanol. Then, step (i) corresponds to a step of providing a methanol synthesis catalyst, preferably in a methanol synthesis unit 600, and step (j) corresponds to the step of contacting said fifth effluent 17 with said methanol synthesis catalyst.

Methanol synthesis is carried out by passing the fifth effluent 17 comprising hydrogen, carbon monoxide and carbon dioxide over a methanol catalyst bed. The reaction of carbon oxides with hydrogen to give methanol is given by the following two equations:

$$CO + 2H_2 \rightleftarrows CH_3OH$$

$$CO_2 + 3H \rightleftarrows CH_3OH + H_2O$$

$$CO_2 + H_2 \rightleftarrows CO + H_2O$$

To have good methanol synthesis yields, it is preferable that the fifth effluent 17 has a composition close to the stoichiometric ratio. The stoichiometric ratio can be defined with the S ratio, where S is defined as it follows: $S = \frac{H_2 - CO_2}{CO + CO_2}$. An S ratio is preferably in the range of 1.0 to 2.5, more preferably in the range of 1.8 to 2.2, even more preferably the S ratio should be at 2.0.

The methanol synthesis process can be operated in an adiabatic reactor or in an isothermal reactor like of instance according to US2017021322. For an adiabatic process, the temperature is the temperature at the inlet of the reactor. For an isothermal reactor, the temperature is the average temperature in the catalyst bed.

The methanol synthesis process can be operated at a temperature between 120°C to 300°C, preferably between 180 and 280° C, even more preferably between 190°C and 250°C. The methanol synthesis process can be operated at a pressure between 1 MPa to 20 MPa, preferably between 5 to 10 MPa, or between 5 and 8 MPa. The GHSV can advantageously range from 0.1 h$^{-1}$ to 50 h$^{-1}$, preferably from 1 h$^{-1}$ to 50 h$^{-1}$.

The methanol synthesis reaction can be performed over a copper-based catalyst like for example a CuO/ZnO/Al$_2$O$_3$ catalyst. Other types of catalysts can be prepared according to WO2017118572 and WO2017118573.

The sixth effluent 21 comprises methanol, but it can also comprise unconverted H$_2$, unconverted CO$_2$, unconverted CO and/or water.

The recovery of crude methanol from the sixth effluent 21 can advantageously be done in a methanol separation unit 700 by condensation, namely by cooling the sixth effluent 21 below the boiling point and separating the methanol as a liquid effluent 23 being the crude methanol. The crude methanol can advantageously be further purified by distillation.

The remaining unreacted gases form a gaseous stream 25 containing H$_2$, CO$_2$ and/or CO and can be optionally recycled to be further converted into methanol. Also, fresh gases can be advantageously incorporated into the methanol synthesis unit 600 via a first line 27 of hydrogen and/or a second line 29 of carbon dioxide and/or a third line 31 of carbon monoxide to adjust the stoichiometric ratio S.

The methanol synthesis reaction is exothermic and although the process can be run adiabatically, it is advantageous to cool the methanol synthesis catalyst to run the process. In that case, a methanol synthesis process should be capable of providing a quantity of usable heat.

As a matter of non-limiting examples, the methanol synthesis process can be according to EP 1 080 059 describing a process wherein methanol is synthesized in a synthesis loop in at least two synthesis stages from a synthesis gas comprising hydrogen and carbon oxides. Another possibility is the use of more than one methanol reactor as described in US 2010/0160694. The methanol synthesis process can also be as disclosed in US 8,629,190 where the synthesis gas is passed through a first, preferably water-cooled reactor, in which a part of the carbon oxides in the gas is catalytically converted to methanol, and the resulting mixture of synthesis gas and methanol vapor is supplied to a second, preferably gas-cooled reactor in series.

It is also advantageous that the synthesis gas used for methanol synthesis is free of sulphur or sulphur-containing compounds, as the catalyst used for methanol production could be irreversibly poisoned through the formation of sulphides. In a preferred embodiment said synthesis gas could optionally be passed through a sulphur trap to remove H$_2$S and/or sulphur-containing species. Said trap could comprise a sorbent material.

Optional reverse water gas shift reaction (steps (k) and (I) of the process)

Optionally, before contacting the fifth effluent 17 with the methanol synthesis catalyst, it is possible to increase the amount of carbon monoxide to form a CO-enriched effluent 19 that can be then directed into the methanol synthesis unit 600. For forming the CO-enriched effluent 19, the fifth effluent is directed from the hydrogen recovery unit 400 into a reverse water gas shift (RWGS) unit 500 that comprises a reverse water gas shift catalyst. Then, step (k) of the process corresponds to the step of providing a reverse water gas shift catalyst and step (I) of the process corresponds to the step of contacting said fifth effluent 17 with said reverse water gas shift catalyst to form the CO-enriched effluent 19. It is noted that said CO-enriched effluent 19 can then be used in step (j) of the process, namely, it can be contacted with the methanol synthesis catalyst.

The reverse water gas shift reaction converts hydrogen and carbon dioxide into water and carbon monoxide according to the following equation: H$_2$+CO$_2 \rightarrow$ H$_2$O + CO

This optional reaction aims to ensure that the composition of the stream entering the methanol synthesis unit 600 is close to the stoichiometric ratio. The RWGS is performed under conditions so that the composition of the CO-enriched effluent 19 exiting the RWGS unit 500 is close to the stoichiometric value. The stoichiometric ratio can be defined with the S ratio, where S is defined as it follows: $S = \frac{H_2 - CO_2}{CO + CO_2}$. An S ratio is preferably in the range of 1.0 to 2.5, more preferably in the range of 1.8 to 2.2, even more preferably the S ratio should be at 2.0.

In a preferred embodiment, the composition at the inlet of the reverse water gas shift unit 500 can be adapted

with $H_2$, $CO_2$, and/or CO makeups. Such makeups are possible via the gaseous stream 25 comprising $H_2$, $CO_2$ and/or CO exiting the methanol separation unit 700 that be recycled into notably the reverse water gas shift unit 500 and/or via the presence of a first line 27 of hydrogen and/or a second line 29 of carbon dioxide and/or a third line 31 of carbon monoxide at the level of the reverse water gas shift unit 500 that are directing fresh gases into the notably the reverse water gas shift unit 500.

The reverse water gas shift reaction can be performed either at a temperature ranging 200°C to 400°C or between 250°C and 350°C, and at a pressure ranging 0.1-10 MPa, and GHSV ranging 1000-50 000 h$^{-1}$. The RWGS reaction is exothermic and it is necessary to cool the gas stream between the high-temperature RWGS stage and the low-temperature RWGS stage to reach low equilibrium levels of CO. Heat exchangers are typically used to cool the gas stream.

In a preferred embodiment, the reverse water gas shift catalyst used for the reverse water gas shift reaction is a copper oxide/zinc oxide catalyst.

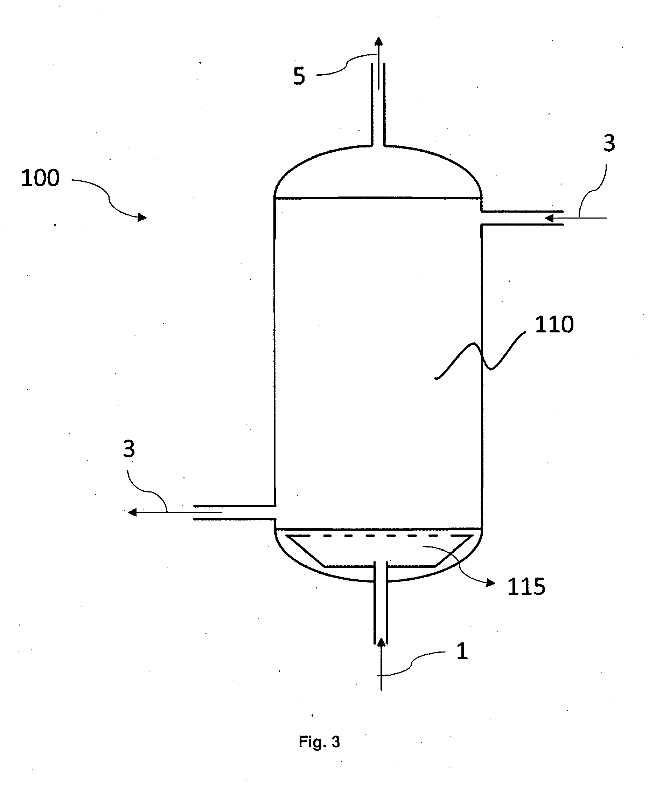

In **figure 3** a trickle bed reactor 110 is presented. In such a trickle bed reactor 110, the conversion of hydrogen sulphide into hydrogen and elemental sulphur is performed simultaneously with the separation of these products. A first stream 1 comprising at least 1 wt.% of $H_2S$ based on the total weight of said first stream and optionally at least 1 wt.% of carbon oxides based on the total weight of said first stream, said carbon oxides being selected from carbon monoxide and/or carbon dioxide, and a second stream 3 comprising a solvent are sent counter-currently and in the bottom of the trickle bed reactor 110. On the top of the reactor, a first effluent 5 comprising a third stream 7 *(i.e., the solvent and elemental sulphur)* and a fourth stream 9 *(i.e., $H_2$, unconverted $H_2S$ if any and carbon oxides when carbon oxides are present in the first stream 1)* is recoveredUniform repartition of the $H_2S$ is ensured via a flow repartition unit 115 in the bottom of the trickle bed reactor 110.

Composition of the various streams and effluents

The **first stream 1** comprises at least 1 wt.% of hydrogen sulphide based on the total weight of said first stream 1, preferably at least 30 wt.%, more preferably at least 50 wt.%, even more preferably at least 75 wt.%, most preferably at least 90 wt.%, even most preferably at least 95 wt.% or at least 99 wt.%, or the first stream comprises only hydrogen sulphide. The first stream 1 can comprise $CH_4$, $H_2O$, and/or one or more hydrocarbons having from 2 to 6 carbons atoms, preferably at a concentration below 10 wt.% based on the total weight of said first stream 1; for example, at a concentration below 5 wt.%.

Depending on the level of the concentration of carbon oxides in the first stream 1, such as carbon monoxide and/or carbon dioxide, it is possible to generate methanol.

When the first stream 1 comprises carbon oxides such as $CO_2$ and/or CO in an amount below 1.0 wt.%, preferably below 0.1 wt.%, even more preferably below 0.01 wt.% based on the total weight of the first stream 1, it is not worth treating the carbon oxides to form methanol. The embodiment that is described in figure 1 can then be advantageously followed.

However, when the first stream 1 comprises carbon oxides in an amount of at least 1.0 wt.% based on the total weight of the first stream 1, or between 1 wt.% and 50 wt.% based on the total weight of the first stream 1, preferably between 5 wt.% and 45 wt.%, more preferably between 10 wt.% and 40 wt.% or between 15 wt.% and 35 wt.%, it is worth to continue the process to generate methanol. The embodiment that is described in figure 2 can then be advantageously followed.

The **first stream 1** can contain other components including sulphur-based components such as thiols, for example, methane thiols, dithiols or thiophenic components. The first stream 1 can originate from an amine gas treatment. It can also originate from any other process treatment in a refinery, e.g. hydrotreatment units, or in the natural gas process. Other components of said first stream 1 could be one or more hydrocarbons, for example, methane, ethane, propane, ethylene, benzene, toluene, xylene and any mixture thereof. With preference, said first stream 1 is a gaseous stream. In a preferred embodiment, said first stream comprises at least 1 mol % of methane and/or ethane and/or propane to at most 50 mol % of methane and/or ethane and/or propane based on the total molar content of the first stream 1, and/or at least 0.1 mol % of ethylene and/or benzene and/or toluene and/or xylene to at most 20 mol % of ethylene and/or benzene and/or toluene and/or xylene based on the total molar content of the first stream 1. To the first stream (1), it can optionally be added the fourth effluent 15 comprising unconverted $H_2S$.

The **second stream 3** comprises a solvent, said solvent comprising one or more organic compounds having a C-N bond. More data about the solvent of the second stream 3 are given in the below section. The second stream 3 can be a mixture of a fresh solvent with the solvent of the third effluent 13 which is recovered according to the process of the disclosure. The losses of solvent are very low in the process. The second stream 3 is therefore mainly constituted of regenerated solvent.

The **first effluent 5** comprises all the effluents exiting the reaction unit 100. Therefore, the first effluent 5 comprises the solvent, elemental sulphur, hydrogen and unconverted hydrogen sulphide if any.

The concentration of solvent can vary from 1 wt.% to 80 wt.% based on the total weight of the first effluent 5, or from 5 wt.% to 80 wt.%, preferably from 10 to 75 wt.%, more preferably from 15 wt.% to 70 wt.%, even more preferably from 20 wt.% to 65 wt.%, most preferably from 25 wt.% to 60 wt.% or from 25 wt.% to 50 wt.%.

The concentration of elemental sulphur $S_8$ in the solvent part of the first effluent 5 can vary from 1 wt.% to 50 wt.% based on the total weight of the first effluent 5, preferably from 5 wt.% to 45 wt.% or from 10 wt.% to 45 wt.%.

The concentration of $H_2$ in the first effluent 5 can vary from 1 wt.% to 50 wt.% based on the total weight of the first effluent 5, preferably from 5 wt.% to 45 wt.% or from 10 wt.% to 45 wt.%.

The concentration of $H_2S$ in the first effluent 5 can vary from 0.01 wt.% to 1.00 wt.% based on the total weight of the first effluent 5, preferably from 0.05 wt.% to 0.95 wt.%, more preferably from 0.10 wt.% to 0.90 wt.%.

The **third stream 7** comprises mainly the liquid part of the first effluent 5. Thus, the third stream comprises the solvent and the elemental sulphur. For example, the third stream 7 is a liquid stream.

The third stream 7 can comprise at least 75 wt.% of solvent based on the total weight of the third stream 7, preferably at least 80 wt.%, even more preferably at least 95 wt.%.

The concentration of elemental sulphur $S_8$ in the third stream 7 can range from 1 wt.% to 75 wt.% based on the total weight of the third stream 7, preferably from 5 wt.% to 70 wt.%, more preferably from 10 wt.% to 65 wt.%. For example, the concentration of elemental sulphur $S_8$ in the third stream 7 can be at least 1 wt.% based on the total weight of the third stream 7, preferably at least 5 wt.%, more preferably at least 10 wt.%. For example, the concentration of elemental sulphur $S_8$ in the third stream 7 can be of at most 75 wt.%, preferably at most 70 wt.%, more preferably at most 65 wt.%.

Traces of disulphide $S_2$ can also be present in the third stream 7, *i.e.* $S_2$ can be present in the third stream 7 at a concentration below 10 wt.% based on the total weight of the third stream 7, preferably below 5 wt.%, more preferably below 1 wt.%.

The **fourth stream 9** comprises mainly the gaseous part of the first effluent 5. Thus, the fourth stream 9 comprises hydrogen and unconverted hydrogen sulphide if any. For example, the fourth stream 9 is a gaseous stream.

The fourth stream 9 can, comprise at least 75 wt.% of $H_2$ based on the total weight of the fourth stream 9, preferably at least 85 wt.%, more preferably at least 90 wt.%.

The fourth stream 9 can also comprise unconverted $H_2S$ at a concentration of less than 10 wt.% based on the total weight of the stream, preferably less than 5 wt.%, more preferably less than 1 wt.%.

**The second effluent 11** is an effluent obtained after having removed the solvent from the third stream 7 in the sulphur recovering unit 300 and comprises the elemental sulphur $S_8$. For example, the second effluent 11 is a liquid stream. The second effluent 11 can also comprise disulphide $S_2$ with possible traces of $H_2S$ and solvent. Once a crystallization step is performed, these impurities ($S_2$, $H_2S$, solvent, and any other sulphur derivatives) can be found in the form of inclusions into the defects of crystals of elemental sulphur. Said crystallization step could be achieved by cooling sulfur to the temperature below melting point, 113°C.

The content of $S_8$ can be of at least 50 wt.% based on the total weight of the second effluent 11, preferably at least 75 wt.%, more preferably at least 80 wt.%, even more preferably at least 95 wt.%, most preferably at least 99 wt.%.

The content of $S_2$ can be below 10 wt.% based on the total weight of the second effluent 11, preferably below 5 wt.%, more preferably below 1 wt.%.

Treatment of the second effluent 11 by distillation and/or by sublimation allows obtaining elemental sulphur $S_8$ without the impurities, such as $S_2$, $H_2S$ or any other sulphur derivatives.

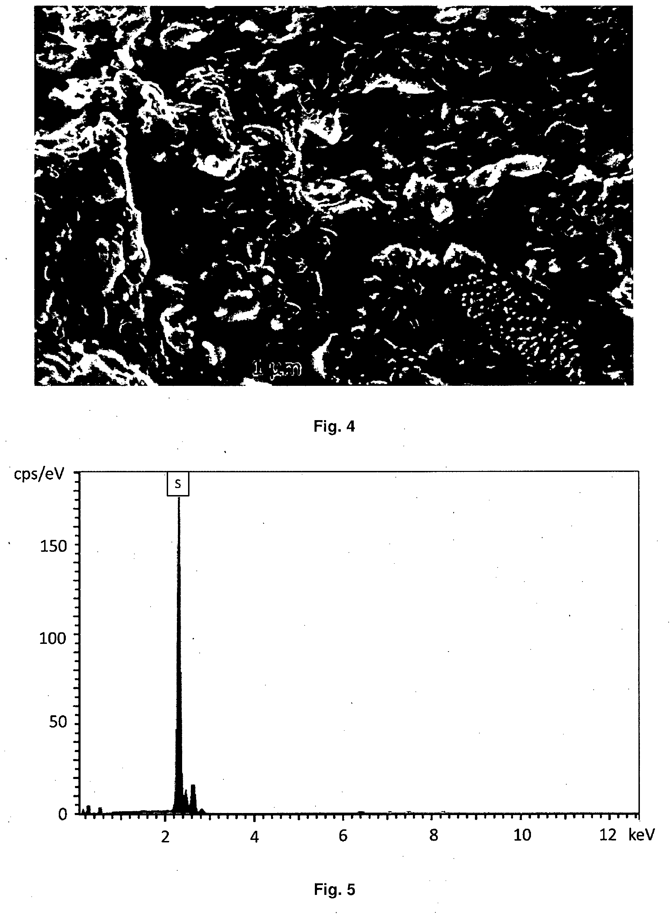

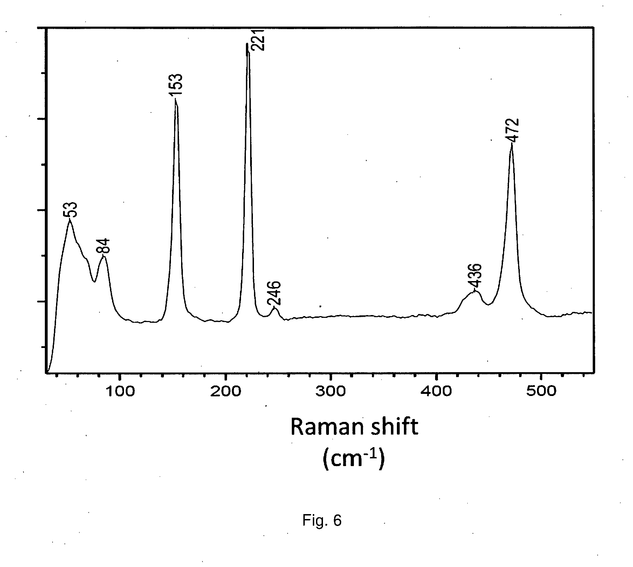

Characterization of the elemental sulphur can be performed using well-known techniques: EDX (see figure 5) or Raman spectroscopy (see figure 6).

The **third effluent 13** comprises mainly the regenerated solvent that can be recovered in the sulphur recovering unit 300. Once regenerated, the solvent does not contain anymore a significant amount of elemental sulphur. The various fractions of each component of the solvent, such as the amount of one or more organic compounds having a C-N bond, and optionally the amount of water and/or the amount of the one or more hydrocarbons and/or the amount of carbon disulphide if any, stay unchanged during the process. The composition of the regenerated solvent is thus the same as the composition of the fresh solvent.

The **fourth effluent 15** comprises mainly the unconverted $H_2S$ that can be recovered in the hydrogen recovering unit 400. For example, the fourth effluent 15 is a gaseous effluent. The fourth effluent 15 can comprise at least 80 wt.% of $H_2S$ based on the total weight of the fourth effluent 15, preferably at least 90 wt.%, more preferably at least 95 wt.%.

The **fifth effluent 17** comprises the $H_2$ produced during the process that can be recovered in the hydrogen recovering unit 400. For example, the fifth effluent 17 is a gaseous effluent. The fifth effluent 17 can comprise at least 50 wt.% of $H_2$ based on the total weight of the fifth effluent 17, preferably at least 60 wt.%, more preferably at least 70 wt.%, even more preferably at least 80 wt.%, most preferably at least 90 wt.%, even most preferably at least 95 wt.%. The fifth effluent 17 can further comprise carbon oxides, such as CO and/or $CO_2$, when said carbon oxides are present in the first stream 1. When carbon oxides are present in the first stream 1, the amount of carbon oxides in the fifth effluent 17 is ranging between 1 wt.% and 50 wt.% based on the total weight of the fifth effluent 17 and the amount of hydrogen is ranging between 50 wt.% and 99 wt.% based on the total weight of the fifth effluent 17.

The **CO-enriched effluent 19** comprises CO, $CO_2$ and $H_2$ at a concentration close to the stoichiometric ratio required for the methanol synthesis.

The **sixth effluent 21** comprises methanol, unconverted $CO_2$, unconverted CO, unconverted $H_2$ and water. The water and the methanol can be separated by condensation via a decrease in the temperature to form the liquid stream 23. The resulting gaseous stream 25 comprises $CO_2$, CO and $H_2$ and can be recycled back to the methanol synthesis unit 600 or to the reverse water gas shift unit 500 when said RWGS unit is present.

Detailed description of the solvent comprised in the second stream 3

The solvent used in the process of the disclosure is preferably a mixture of water with one or more hydrocarbons and one or more organic compounds comprising a C-N bond. The one or more organic compounds comprising a C-N bond can be an amine. Any amine-based reagent capable of selectively absorbing hydrogen sulphide may be used. "Selectively absorbing hydrogen sulphide" refers to the preferential absorption of hydrogen sulphide from the feed gas, while minimizing the absorption of any other components of or contaminants in the feed gas. Thus, in the preferred embodiment, the amine-based reagent is selected to absorb as much of the hydrogen sulphide from said first stream (1) as possible. It is not excluded that such amine simultaneously absorbs and removes as little as possible of the other components of or contaminants in the feed gas, such as carbon dioxide. In other words, the hydrogen sulphide is preferentially absorbed to the exclusion of other components of the feed gas. In the preferred embodiment, the amine allows absorbing high quantities of hydrogen sulphide fraction, preferably at least about 90 percent hydrogen sulphide by weight. Any amine-based reagent capable of "selectively absorbing hydrogen sulphide" may be used, wherein the hydrogen sulphide is absorbed while minimizing the absorption of any other components of the feed gas. For instance, the amine-based reagent may comprise monoethanolamine (MEA), diethanolamine (DEA), diglycolamine (DGA), diisopropanolamine (DIPA), methyldiethanolamine (MDEA), triethanolamine (TEA), UCARSOL™, SELEXOL™ and mixtures thereof. UCARSOL™ and SELEXOL™ are trademarks of The Dow Chemical Company and refer to a group of amine solvents manufactured thereby.

In the preferred embodiment, the amine-based reagent comprises a Flexsorb™ amine-based reagent or Sulfinol-M™. Flexsorb™ is a trade-mark of Exxon Corporation and refers to a group of solvents comprising amines and manufactured thereby by a proprietary process, including FLEXSORB SE™ and FLEXSORB SE PLUS™. Sulfinol™ is a trade-mark of Shell Corporation and refers to a group of solvents comprised of amines and manufactured thereby by a proprietary process, including Sulfinol-D™, Sulfinol-M™ and Sulfinol-X™.

The solvent also preferably comprises one or more hydrocarbons. The following compounds can be used: 1,2-dichloroethane ($C_2H_4Cl_2$), carbon tetrachloride ($CCl_4$), nitrobenzene ($C_6H_5NO_2$), chloroform ($CHCl_3$), cyclohexane ($C_6H_{12}$), aniline ($C_6H_5NH_2$), pyridine ($C_5H_5N$), *m*-xylene [$(CH_3)_2C_6H_4$], *p*-xylene [$(CH_3)_2C_6H_4$], *o*-xylene, [$(CH_3)_2C_6H_4$], toluene ($CH_3C_6H_5$), benzene ($C_6H_6$), chlorobenzene ($C_6H_5Cl$), 1,2-dibromoethane ($C_2H_4Br_2$), dichloromethane, diphenyl ether ($C_{12}H_{10}O$), biphenyl ($C_{12}H_{10}$), any C5 to C10 alkanes, any C5-C10 alkenes, and any mixture thereof. Toluene is the preferred hydrocarbon.

In the particular case of said step (f) being performed by distillation or by steam distillation, said solvent may comprise $CS_2$. The presence of $CS_2$ is particularly advantageous because sulphur has a high solubility in this solvent.

The solvent used in the process of the disclosure is preferably a mixture of water with one or more hydrocarbons and one or more organic compounds comprising a C-N bond. Those various components may sometimes be difficult to mix. The mixing of water with one or more hydrocarbons and with one or more organic compounds comprising a C-N bond to produce the solvent of the second stream 3 can be done before the use of the second stream 3. This mixing can be done in a dedicated stirred tank with strong agitation. In this case, a large quantity of solvent may be agitated while only part of the mixed solvent is subtracted for performing the process. Other processes such as recirculation pump may also be used.

The solvent used in the process of the disclosure is preferably a mixture of water with one or more hydrocarbons and one or more organic compounds comprising a C-N bond. In a preferred embodiment, the weight ratio in said solvent of water and one or more organic compounds comprising a C-N bond versus the hydrocarbons ranges between 100/1 to 1/100, preferably between 90/1 to 10/100, more preferably between 80/1 to 20/100.

Detailed description of step (f), corresponding to the removal of the solvent

**In a first implementation,** said step (f) of removing the solvent from the third stream is performed by distillation. With preference, said distillation is carried out at a temperature of above 150°C. For example at a temperature from above 150°C to below 450°C, with preference at temperature ranging between 155°C or above and 445°C or below, or between 160°C or above and 440°C or below. The choice of the temperature for the distillation is dependent of the solvent used since the solvent must be gaseous while a the same time the element sulfur must be liquid. For example, said distillation is carried out at a pressure ranging between 0.0001 MPa and a pressure of 10 MPa, more preferably between 0.0005 and 5 MPa, even more preferably between 0.001 MPa and 1 MPa. Advantageously, the solvent of the second stream further comprises carbon disulphide, preferably at a concentration ranging from 1 to 90 wt.% based on the total weight of said second stream, more preferably from 5 to 80 wt.%, even more preferably from 10 wt.% to 70 wt.%, most preferably from 15 wt.% to 60 wt.%, even most preferably from 20 wt.% to 50 wt.%.

**In a second implementation,** said step (f) of removing the solvent from the third stream is performed by steam distillation. For example, said steam distillation comprises the following sub-steps: (f1) stripping the third stream with steam to recover the third effluent and (f2) recovering the second effluent by decantation and/or filtration. Optionally, a sixth effluent comprising hydrogen sulphide is recovered from step (f1). When the sixth effluent is recovered, the process advantageously comprises the step of directing said sixth effluent into the first stream provided in step (a) and/or used in step (d). For example, the sub-step (f1) is carried out at a temperature of at least 105°C, preferably at least 110°C, more preferably at least 115°C, more preferably at least 120°C or at least 130°C. Advantageously, the solvent of the second stream further comprises carbon disulphide, preferably at a concentration ranging from 1 to 90 wt.% based on the total weight of said second stream, more preferably from 5 to 80 wt.%, even more preferably from 10 wt.% to 70 wt.%, most preferably from 15 wt.% to 60 wt.%, even most preferably from 20 wt.% to 50 wt.%.

**In a third implementation,** said step (f) of removing the solvent from the third stream is performed by extraction over carbon dioxide or sulphur dioxide. For example, said extraction over carbon dioxide or sulphur dioxide comprises the following sub-steps: (f1) addition of carbon dioxide or sulphur dioxide on the third stream to form a saturated solution, (f2) heating the saturated solution at a temperature of at least 100°C and (f3) recovering the second effluent, the third effluent, and optionally a carbon dioxide stream or a sulphur dioxide stream.

The sub-step (f1), being the addition of $CO_2$ or $SO_2$ on the third stream 7 comprising the solvent and the elemental sulphur, is the generation of a saturated third stream. The addition of $CO_2$ generates hydrocarbonates and carbonates. The addition of $SO_2$ generates hydrosulphites and sulphites. These (hydro)carbonates or (hydro)sulphites saturates the third stream 7 and leads to the precipitation of elemental sulphur $S_8$. The solution saturated with (hydro)carbonates or (hydro)sulphites can advantageously be regenerated via heating in the sub-step (f2). Heating will form a carbon dioxide stream or a sulphur dioxide stream that can be recovered in sub-step (f3) along with the third effluent 13 comprising the solvent and the second effluent 11 comprising the elemental sulphur.

With preference, the sub-step (f2) is performed at a temperature of at least 110°C, more preferably at least 120°C or at least 130°C.

With preference, the sub-step (f3) is performed by decantation or filtration. Advantageously, the solvent of the second stream is devoid of carbon disulphide.

**In a fourth implementation,** said step (f) of removing the solvent from the third stream is performed by extraction over an adsorbent. For example, said extraction over an adsorbent comprises the following sub-steps: (f1) recovering of the third effluent by adsorption on an adsorbent of the third stream, and (f2) recovering of the second effluent by heating said adsorbent at a temperature of at least 100°C. With preference, said adsorbent is one or more selected from copper oxide, zinc oxide, amorphous aluminum, zeolite, hydrated alumina, crystalline boehmite, pseudo-boehmite, and gibbsite. For example, said adsorbent is blended with a binder and/or a diluent. Advantageously, the sub-step (f2) is performed at a temperature of at least 110°C, preferably at least 120°C, more preferably at least 130°C, even more preferably at least 140°C. Advantageously, the solvent of the second stream is devoid of carbon disulphide.

Detailed description of the catalyst composition provided at step (c)

The catalyst composition provided at step (c) is provided in the form of a catalytic bed. For example, the catalytic bed used in the present disclosure can be in the form of a fixed bed.

With preference, the catalyst composition is constituted with a catalyst, and the catalyst has a spherical shape.

The catalyst composition provided at step (c) can comprise as a catalyst one or more selected from alkali metal sulphide, alkali earth metal sulphide, transition metal sulphide, alkali metal hydrosulfite, alkali earth metal hydrosulfite, transition metal hydrosulfite, alkali metal polysulphide, alkali earth metal polysulphide, transition metal polysulphide. With preference, the catalyst composition provided at step (c) comprises as a catalyst at least one transition metal sulphide.

The catalyst composition is constituted with a catalyst, and the catalyst can be dispersed on a carrier. Alternatively, the catalyst composition does not comprise a carrier.

For example, the catalyst can comprise $FeS$, $FeS_2$, $Fe_3S_4$, $Fe_9S_{11}$, $[Fe_2S_{12}]^{2-}$, $[FeS_{12}]^{2-}$, $Na_2S$, $Na_2S_2$, $Na_2S_3$, $Na_2S_4$, $Na_2S_5$, $Cp_2TiS$, $Cp_2TiS_3$, $Cp_2TiS_5$, $Cp_2ZrS$, $Cp_2ZrS_3$, $Cp_2ZrS_5$, $Cp_2VS$, $Cp_2VS_3$, $Cp_2VS_5$, $Cp_2HfS$, $Cp_2HfS_3$, $Cp_2HfS_5$, $Cp_2ThS$, $Cp_2ThS_3$, $Cp_2ThS_5$, $MoS_2$, $[MoS_4]^{2-}$, $[MoS_9]^{2-}$, $[Mo_2S_{12}]^{2-}$, $[Mo_2S_8]^{2-}$, $[Mo_2S_{10}]^{2-}$, $[Mo_3S_9]^{2-}$, $[Mo_3S_{14}]^{2-}$, $[FeMoS_{10}]^{2-}$, $[NiS_8]^{2-}$, $[CuS_4]^-$, $[Cu_3S_{18}]^{3-}$, $[Cu_3S_{12}]^{3-}$, $[Cu_2S_{20}]^{4-}$, $[Cu_4S_{12}]^{2-}$, $[Cu_4S_{13}]^{2-}$, $[Cu_4S_{14}]^{2-}$, $[Cu_4S_{15}]^{2-}$, $[Cu_6S_{17}]^{2-}$, $ZnS$, $[Zn(SR)_4]^{2-}$, $[Zn(SR)_2S_4]^{2-}$, $[Z_n(SR)_2S_5]^{2-}$, $[ZnS_8]^{2-}$, $[ZnS_{10}]^{2-}$, $[ZnS_{12}]^{2-}$, $CdS$, $[Cd(SR)_4]^{2-}$, $[Cd(SR)_2S_4]^{2-}$, $[Cd(SR)_2S_5]^{2-}$, $[CdS_8]^{2-}$, $[CdS_{10}]^{2-}$, $[CdS_{12}]^{2-}$, $MnS$, $[Mn(SR)_4]^{2-}$, $[Mn(SR)_2S_4]^{2-}$, $[Mn(SR)_2S_5]^{2-}$, $[MnS_8]^{2-}$, $[MnS_{10}]^{2-}$,

$[MnS_{11}]^{2-}$, $[MnS_{12}]^{2-}$, where Cp corresponds to cyclopentadiene, with corresponding polysulphide and where R corresponds to a hydrocarbon radical having at least one carbon, preferably two and up to 25 carbon atoms; or any mixture thereof.

The transition metal sulphide is preferably selected from sulphides of chromium, molybdenum, tungsten, manganese, iron, cobalt, nickel and copper and also mixtures thereof. The transition metal sulphide should preferably be selected from sulphides of iron, cobalt, nickel, copper and also mixtures of these sulphides, iron sulphide being very particularly preferred. Certain metallic elements found to form suitable lower metal sulphides include those selected from the group of metals consisting of iron (Fe), nickel (Ni), cobalt (Co), copper (Cu), cadmium (Cd), manganese (Mn), lead (Pb), tin (Sn), titanium (Ti), zirconium (Zr), hafnium (Hf), vanadium (V), molybdenum (Mo), zinc (Zn), thorium (Th), selenium (Se), tellurium (Te), potassium (K), caesium (Cs), rubidium (Rb) and sodium (Na). Among these metals, iron and nickel are the preferred metal of the metal sulphides that are useful in the process. Examples of some of the lower forms of the metal sulphides include CdS, FeS, $Fe_7S_8$, NiS, $Ni_2S_3$, CoS, $Co_9S_8$, $Sn_3S_4$, $Na_2S$, $Na_2S_2$ and $Na_2S_3$. Both lead and tin may decompose to the metallic form which is convertible into the sulphide form. Non-limiting examples of some of the higher forms of the metal sulphides that can be used in the process include $FeS_2$, $NiS_2$, $CoS_2$, $CuS_2$, PbS, $Sn_2S_3$, and $Na_2S_Z$ (wherein Z=3 or 4 or 5 or 6). The preferred metal sulphides for use in the process are the sulphides of iron or nickel or cadmium.

The metal sulphide of the process may be in any form that suitably allows its use in the reactors or reaction zones of the process. For example, the metal sulphide may be in the form of small particles comprising the metal sulphide that can be easily dispersed in the solvent or may be in the form of deposits on the static meshes installed along the reactor. Also, the metal sulphide may be composited with an inert material, such as the inorganic metal oxides of alumina, silica, titania and the like, or the metal sulphide may be composited with reactive components, or the metal sulphide may be composited with both inert material and reactive components, and then shaped into formed particles that may be used in the reactors or reaction zones of the process.

The catalyst of the catalyst composition provided at step (c) can also comprise stainless steel, for example, at a content ranging between 1 wt.% and 90 wt.% based on the total weight of said catalyst composition, with preference between 5 wt.% and 80 wt.% or between 10 wt.% and 70 wt.%. For example, stainless steel can be one or more selected from austenitic stainless steel, ferritic stainless steel, duplex stainless steel, martensitic, stainless steel, precipitation hardening stainless steel or any mixture thereof.

The fixed bed reactor has a catalyst bed comprising stainless steel, wherein said stainless steel is preferably one or more selected from austenitic stainless steel, ferritic stainless steel, duplex stainless steel, martensitic, stainless steel, precipitation hardening stainless steel or any mixture thereof. For example, the stainless steel is packed stainless steel, preferably having a packed surface of at least 50 $m^2/m^3$. For example, stainless steel is in the form of Raschig rings.

The catalyst bed or the catalyst can also be made of stainless steels. None limiting examples include austenitic sainless steel (for instance 301, 301L, 301LN; 302HQ; 303, 303Se; 304, 304L, 304H; 310, 310S, 310H; 316, 316L, 316H; 321, 321H, 347; 253MA (S30815); 904L), ferritic stainless steel (for instance AtlasCR12, AtlasCR12Ti, 409, 430, 430F, F18S / 439, F20S, 304 / 304L, F18MS / 444) duplex stainless steel, (for instance 2101, 2304, 2205, 2507, 2507Cu) martensitic stainless steel (for instance 410, 416, 420, 431, 440A, 440B, 440C); precipitation hardening stainless steel (for instance 630).

Detailed description of step (d)

The reaction performed in step (d) can be performed in a trickle bed reactor, a batch reactor or in a plug flow reactor or in a continuous stirred-tank reactor or in a recycle reactors (i.e. a plug flow reactor with a recycle loop) or in a slurry bubble reactor.

For instance, when the reaction of step (d) is performed in a trickle bed reactor, the reaction is performed at a temperature ranging between 5 to 80°C preferably from 10 to 50°C and/or at a pressure ranging from 0.1 to 5 MPa, preferably 0.1 to 1 MPa and/or the WHSV of the second stream 3 ranges from 0.01 $s^{-1}$ to 500 $s^{-1}$ in step (d) and/or the GHSV of the first stream 1 ranges from 0.01 $s^{-1}$ to 500 $s^{-1}$ in step (d).

Detailed description of step (h)

The fourth stream 9 comprises hydrogen ($H_2$) and unconverted hydrogen sulphide ($H_2S$) if any. It is advantageous that the process further comprises a step (h) of separating the fourth stream 9 into a fourth effluent 15 comprising the unconverted hydrogen sulphide if any and into a fifth effluent 17 comprising hydrogen and carbon oxides, such as $CO_2$ and/or CO when carbon oxides are present in the first stream 1.

In the optional step (h), the unconverted $H_2S$ is separated from the $H_2$ produced. This type of separation can be done by any known technics. In particular, the optional step (h) can be performed by steam stripping, distillation or acidification of the solution.

In particular, an amine can be used to absorb $H_2S$. In that case, it is assumed, without willing to be bound by any theory that sulphur interacting with amine solution in presence of $H_2S$ is an acid-base reaction followed by reversible formation of polysulphide according to:

$$2\ RNH_2 + H_2S \rightarrow (RNH_2)_2{}^*H_2S$$

$$(RNH_2)_2{}^*H_2S + S_x \rightarrow (RNH_2)_2{}^*H_2S_{x+1}$$

wherein R= $HOCH_2CH_2$- or another carbon, hydrogen and optionally oxygen and/or nitrogen-comprising radical.

As a non-limiting example, sulphur can be recovered according to the method described in US2890931. Treatment of the amine used to adsorb $H_2S$ at step (h) can be done with steam at a temperature below 150°C. This allows the recovery of a purified amine absorber solution and $H_2S$. The role of hot steam is to reverse the reactions:

$$(RNH_2)_2{}^*H_2S_{x+1} \rightarrow (RNH_2)_2{}^*H_2S + S_x$$

$$(RNH_2)_2{}^*H_2S \rightarrow 2\ RNH_2 + H_2S\uparrow$$

Alternatively, another option for the separation of unconverted $H_2S$ is via acidification with $CO_2$ or $SO_2$. A non-limiting example of solution acidification can be found in US4033410. In the particular case of a polar solvent being an amine, treatment with $CO_2$ or $SO_2$ results in the formation of hydrocarbonates, carbonates, or hydrosulphites, sulphites, respectively according to:

$$RNH_2 + H_2O + CO_2 \rightarrow RNH_2{}^*H_2CO_3$$

$$2RNH_2 + H_2O + CO_2 \rightarrow (RNH_2)_2{}^*H_2CO_3$$

$$RNH_2 + H_2O + SO_2 \rightarrow RNH_2{}^*H_2SO_3$$

$$2RNH_2 + H_2O + SO_2 \rightarrow (RNH_2)_2{}^*H_2SO_3$$

The reactions mentioned remove sulphur from the amine solution in the form of solid precipitate and result in the release of $H_2S$ bound to an amine. Forming hydrocarbonates, carbonates, hydrosulphites, and sulphites could be thermally treated to release $CO_2$ or $SO_2$ and to recover a purified amine absorber solution.

Yet another option is distillation to recover a purified amine absorber solution and molecular sulphur in the form of solid or liquid, depending on the temperature applied for distillation.

**Analytical methods**

The $CO_2$ content in the various stream of the process that can be determined by any means known in the art. Preferably it can be determined with the help of gas chromatography (GC) with a calibrated TCD (Thermal Conductivity Detector). A GCMS with calibrated TIC (Total Ion Current) detector could also be used.

The sulphur content in solvent or the residual sulphur content can be determined for instance via X-Ray Fluorescence (XRF), or by a GC with SCD (Sulphur Chemiluminescence Detector), or by GC with TCD.

**Gas chromatography (GC)** was done using a Micro-GC Agilent 490 equipped with Molesieve 5A, PoraPLOT Q, and Hayesep A columns and a TCD detector

**X-ray fluorescence (XRF)** spectroscopy using an Orbis Micro-EDXRF spectrometer equipped with a Rh source (15 kV, 500 $\mu$A) and a silicon drift detector has been used to determine the content of the sulphur.

**Scanning Electron Microscopy (SEM) and energy dispersive X-ray (EDX) spectroscopy** were respectively used to image the catalyst surface and analyse elemental composition of the sample. This was studied using a SUPRA 35 VP (Carl Zeiss) scanning electron microscope. The working distance was 8-8.3 mm, the accelerating voltage was 3 kV, probe current was 9.6*10-5 A, emission current was 10 $\mu$A, extraction voltage was 6.96 kV.

Elemental sulphur was measured using **Raman spectrometry.** The Raman spectrum was collected on a Jobin Yvon Labram 300 confocal Raman spectrometer coupled to an optical microscope (objective 50x) and a CCD detector. A 532 nm wavelength laser was used, and spectra were accumulated 3 times for 60 s each. The power applied to the sample did not exceed 20 mW upon measurement.

**Examples**

Example 1

A sample of 8 g of powdery CdS was immersed in the mixture of 57 mL of water and 3 mL of monoethanolamine (5 wt.% of MEA) in a stainless-steel reactor equipped with a mechanical stirrer. The feed comprising 26 vol.% of nitrogen, 66 vol.% of methane and 8 vol.% of $H_2S$ was bubbled through the liquid at a rate of 3.4 L/h at a pressure of 0.2 MPa, 25°C, and with a stirring rate of 600 rpm. The effluent of the reactor was analyzed using micro-GC.

Example 2

A sample of 8 g of powdery CdS was immersed in the mixture of 57 mL of water and 3 mL of monoethanolamine in a stainless-steel reactor equipped with a mechanical stirrer. The feed comprising 1 vol.% of helium, 96 vol.% of methane and 3 vol.% of $H_2S$ was bubbled through the liquid at a rate of 2 L/h at a pressure of 0.2 MPa, 40°C, and with a stirring rate of 600 rpm. The effluent of the reactor was analyzed using micro-GC.

Example 3

A sample of 8 g of powdery CdS was immersed in the mixture of 57 mL of water, 20 mL of toluene and 3 mL of monoethanolamine in a stainless-steel reactor equipped with a mechanical stirrer. The feed comprising 26 vol.% of nitrogen, 66 vol.% of methane and 8 vol.% of $H_2S$ was bubbled through the liquid at a rate of 3.4 L/h at a pressure of 0.2 MPa, 40°C, and with a stirring rate of 600 rpm. The effluent of the reactor was analyzed using micro-GC.

| | Example 1 | Example 2 | Example 3 |
|---|---|---|---|
| Total volume of $H_2S$ passed, L | 5.92 | 1.49 | 5.67 |
| Total amount of $H_2$ produced, L | 0.57 | 1.21 | 0.66 |
| Conversion, % | 9.6 | 81.2 | 11.6 |

In the examples provided the formed sulphur is dissolved in the liquid phase or adsorbed on the catalyst surface in the form of deposits (see **figures 4** and **5**). Dissolved sulphur could be removed by distillation methods, whereas solid sulphur could be washed out by any solvent and filtered off or dissolved and removed by distillation.

Example 4 - Sulphur extraction

A 60 mL aliquot of the sample comprising water-MEA solution was isolated from the reactor. Toluene was added to have a concentration of 5 wt.% of toluene in the distillation mixture. The distillation mixture contained 17,320 wt. ppm of sulphur as determined from XRF analysis. It was distilled under reduced pressure of 0.002 MPa and temperature of 80 °C as measured inside the oil bath. The total mass of sulphur crystals collected at the bottom of the flask is 0.59 g.

Example 5 - Sulphur extraction

A 60 mL aliquot of the sample comprising water-MEA solution was isolated from the reactor. Toluene was added to have a concentration of 5 wt.% of toluene in the distillation mixture. The distillation mixture contained 10,500 wt. ppm. of sulphur as determined from XRF analysis. It was distilled under reduced pressure of 0.002 MPa and temperature of 80 °C as measured inside the oil bath. The total mass of sulphur crystals collected at the bottom of the flask is 0.1 g.

Example 6 - Sulphur extraction

A 60 mL aliquot of the sample comprising water-MEA solution and 15,240 wt. ppm of sulphur as determined from XRF analysis was used for sulphur extraction. The aliquot was transferred into a thick-glass experimental setup comprising two flasks, porous glass filter, and refrigeration system suitable for keeping constant temperature of -25°C for $SO_2$ condensation purposes. 100 mL of sulfur dioxide (Matheson Gas Products, 99.98%) was condensed into one of the flasks, then the refrigeration system was removed and pressure in the system was kept at the level of 300 kPa.

**EP 4 180 386 A1**

The SO$_2$ sample was transferred to the aliquot isolated from the reactor and virgorously stirred. After settling down, the lighter aqueous phase was decanted and heavier SO$_2$ phase was transferred into separate flask. The flask was disconnected from the system, placed in a water bath at 40°C and pressure was slowly released through a caustic trap until no SO$_2$ left. Solid sulfur crystals were collected at the bottom of the flask, m = 0.23 g.

Example 7 - CO$_2$ hydrogenation into methanol

The CO$_2$ hydrogenation into methanol was performed on a Cu/LaCrO$_3$ (7 wt.% Cu) catalyst. The catalyst was prepared by the wet-impregnation method. First, LaCrO$_3$ was prepared as described elsewhere (Open Journal of Inorganic Non-Metallic Materials, 2013, 3, 37). Then, copper nitrate was dissolved in distilled water under intense stirring and mixed with appropriate amount of LaCrO$_3$. Obtained slurry was heated to 90 °C until complete evaropation of water. The solid residue was dried at 120 °C for 24 h and was subsequently calcined at 350 °C for 3 h.
The methanol synthesis reaction was carried out in a fixed-bed stainless steel reactor, charged . with a 250-350 mesh of the catalyst. Prior to the reaction, the catalyst (3 g) was pre-treated with N$_2$/H$_2$ (90/10 vol.) mixture at 350 °C for 2 h under atmospheric pressure. Then the temperature was reduced to 270 °C, back pressure flow controller set up to 20 MPa and the feed mixture CO$_2$/H$_2$ (3:1) was introduced. A space velocity (GHSV) was kept around of 2000 h$^{-1}$. The products were analyzed by GC equipped with a TCD detector. x(CO$_2$) = 7.5%, S(CH$_3$OH) = 54.3%, S(CO) = 21.2%, S(CH$_4$) = 7.8%.

**Claims**

1. Process for the continuous conversion of hydrogen sulphide into hydrogen and elemental sulphur, the process comprising the following steps:

   a) providing a first stream (1) comprising at least 1 wt.% of hydrogen sulphide based on the total weight of said first stream (1);
   b) providing a second stream (3) comprising a solvent, said solvent comprising one or more organic compounds having a C-N bond wherein the content of the one or more organic compounds having a C-N bond in the second stream (3) is ranging from 1 to 90 wt.% based on the total weight of said second stream (3);
   c) providing a catalyst composition;
   d) putting in contact said first stream (1) with said second stream (3) and with said catalyst composition under reaction conditions comprising a temperature ranging between 0°C and 100°C and a pressure ranging between 0.01 MPa and 10.0 MPa, to at least partially convert hydrogen sulphide into a first effluent (5) comprising the solvent, elemental sulphur, hydrogen and unconverted hydrogen sulphide if any;
   e) recovering said first effluent (5) and performing a separation on said first effluent (5) to provide at least a third stream (7) comprising the solvent and elemental sulphur;

   said process is **characterized in that** it further comprises the step (f) of removing the solvent from the third stream (7) to recover a second effluent (11) comprising elemental sulphur and a third effluent (13) comprising the solvent, wherein said step (f) is performed by a distillation selected from a distillation or steam distillation, or is performed by an extraction selected from an extraction over an adsorbent or an extraction over carbon dioxide or an extraction over sulphur dioxide.

2. The process according to claim 1, **characterized in that** the first stream (1) further comprises at least 1 wt.% of carbon oxides based on the total weight of said first stream (1), said carbon oxides being selected from carbon monoxide, carbon dioxide and any mixture thereof, and the first effluent (5) of step d) further comprises carbon oxides, and/or **in that** the one or more organic compounds having a C-N bond are one or more selected from diethanolamine, monoethanolamine, methyldiethanolamine, diisopropanolamine, digycolamine and any mixture thereof.

3. The process according to claim 1 or 2, **characterized in that** the second stream (3) further comprises, based on the total weight of the second stream (3)

   - water at a content ranging from 1 to 50 wt.%; and/or
   - carbon disulfide at a content ranging from 1 to 90 wt.%; and/or
   - one or more hydrocarbons at a content ranging from 1 to 50 wt.%.

4. The process according to any one of claims 1 to 3, **characterized in that** the content of the one or more organic compounds having a C-N bond in the second stream (3) is ranging from 1 to 50 wt.% based on the total weight of said second stream (3); **in that** the second stream (3) further comprises one or more hydrocarbons at a content ranging from 1 to 50 wt.% based on the total weight of said second stream (3) and **in that** the step (f) of removing the solvent from the third stream (7) is performed by distillation; with preference, said distillation is carried out at a temperature ranging between above 150°C and below 450°C, and/or the second stream (3) further comprises from 5 to 45 wt.% of water based on the total weight of said second stream (3).

5. The process according to any one of claims 1 to 3, **characterized in that** the second stream (3) further comprises one or more hydrocarbons at a content ranging from 1 to 50 wt.% based on the total weight of said second stream (3) and **in that** the step (f) of removing the solvent from the third stream (7) is performed by steam distillation; wherein the steam distillation comprises the following sub-steps: (f1) stripping the third stream (7) with steam to recover the third effluent (13) and (f2) recovering the second effluent (11) by decantation and/or filtration.

6. The process according to any one of claims 1 to 3, **characterized in that** said step (f) of removing the solvent from the third stream (7) is performed by extraction over carbon dioxide or by extraction over sulphur dioxide; wherein the extraction over carbon dioxide or the extraction over sulphur dioxide comprises the following sub-steps: (f1) addition of carbon dioxide or sulphur dioxide on the third stream (7) to form a saturated solution, (f2) heating the saturated solution at a temperature of at least 100°C and (f3) recovering the second effluent (11) and the third effluent (13); with preference, the second stream (3) further comprises from 5 to 50 wt.% of water based on the total weight of said second stream (3).

7. The process according to any one of claims 1 to 6, **characterized in that** the second stream (3) further comprises one or more hydrocarbons, wherein the one or more hydrocarbons are selected from 1,2-dichloroethane, carbon tetrachloride, nitrobenzene, chloroform, cyclohexane, aniline, pyridine, m-xylene, p-xylene, o-xylene, toluene, benzene, chlorobenzene, 1,2-dibromoethane, dichloromethane, diphenyl ether, biphenyl, any C5 to C10 alkanes, any C5-C10 alkenes, and any mixture thereof; with preference, the one or more hydrocarbons are or comprise toluene.

8. The process according to any one of claims 1 to 3, **characterized in that** said step (f) of removing the solvent from the third stream (7) is performed by extraction over an adsorbent selected from copper oxide, zinc oxide, amorphous aluminum, zeolite, hydrated alumina, crystalline boehmite, pseudo-boehmite, gibbsite or any mixture thereof; wherein the extraction over an adsorbent comprises the following sub-steps: (f1) recovering of the third effluent (13) by adsorption on an adsorbent of the third stream (7), and (f2) recovering of the second effluent (11) by heating said adsorbent at a temperature of at least 100°C; with preference, the adsorbent is blended with a binder and/or a diluent.

9. The process according to any one of claims 1 to 8, **characterized in that** said process further comprises a step (g) of recycling said third effluent (13) at step (d); and/or said step (d) and said step (e) are performed simultaneously.

10. The process according to any one of claims 1 to 9, **characterized in that** the step (e) of recovering said first effluent (5) and performing a separation on said first effluent (5) further provides at least a fourth stream (9) comprising hydrogen, unconverted hydrogen sulphide if any and carbon oxides when carbon oxides are present in said first stream (1); with preference, said process further comprises a step (h) of performing a separation on said fourth stream (9) to provide at least a fourth effluent (15) comprising the unconverted hydrogen sulphide if any.

11. The process according to claim 10, **characterized in** said process further comprises a step (h) of performing a separation on said fourth stream (9) to provide at least a fifth effluent (17) comprising hydrogen and carbon oxides when carbon oxides are present in said first stream (1); optionally, said process further comprises a step (k) of providing a reverse water gas shift catalyst and a step (l) of contacting said fifth effluent (17) with said reverse water gas shift catalyst to form a CO-enriched effluent (19) comprising at least hydrogen, carbon dioxide and carbon monoxide.

12. The process according to claim 11, **characterized in that** said process further comprises a step (i) of providing a methanol synthesis catalyst and step (j) of contacting said fifth effluent (17) and/or said CO-enriched effluent (19) if formed with said methanol synthesis catalyst to produce a sixth effluent (21) comprising at least methanol.

13. The process according to any one of claims 1 to 12, **characterized in that** the catalyst composition provided at step (c) comprises a catalyst being one or more selected from alkali metal sulphide, alkali earth metal sulphide, transition metal sulphide; alkali metal hydrosulfite, alkali earth metal hydrosulfite, transition metal hydrosulfite, alkali metal polysulphide, alkali earth metal polysulphide, transition metal polysulphide; with preference, the catalyst composition of provided at step (c) comprises a catalyst being one or more transition metal sulphides.

14. Installation to perform the process according to any one of claims 1 to 13, said installation comprising

- a reaction unit (100),
- a separation unit (200) and
- a sulphur recovering unit (300),

wherein the reaction unit (100), the separation unit (200) and the sulphur recovering unit (300) are fluidically connected in series, the separation unit (200) being downstream of the reaction unit (100) and upstream of the sulphur recovering unit (300),

said installation is **characterized in that** said sulphur recovering unit (300) comprises at least one distillation apparatus, or at least one stripping apparatus, or at least one extraction apparatus, or least one adsorbent; with preference, said installation further comprises a line directed between the sulphur recovering unit (300) and the reaction unit (100) to direct a third effluent (13) from the sulphur recovering unit (300) to the reaction unit (100).

15. Installation according to claim 14, **characterized in that** said installation further comprises a hydrogen recovering unit (400), said hydrogen recovering unit (400) being fluidically connected in series to the separation unit (200) and being downstream of said separation unit (200); with preference, said installation further comprises a methanol synthesis unit (600), said methanol synthesis unit (600) being fluidically connected in series to the hydrogen recovering unit (400) and being downstream of said hydrogen recovering unit (400).

Fig. 1

Fig. 2

Fig. 3

**Fig. 4**

**Fig. 5**

Raman shift
(cm$^{-1}$)

Fig. 6

Europäisches
Patentamt

European
Patent Office

Office européen
des brevets

# EUROPEAN SEARCH REPORT

**Application Number**

EP 21 31 5243

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2018/189637 A1 (SABIC GLOBAL TECHNOLOGIES BV [NL]; CHINTA SIVADINARAYANA [SA]) 18 October 2018 (2018-10-18) * claims 1-20 * * paragraphs [0053] - [0056] * * figures 1, 2a, 2b * | 1-15 | INV. C01B17/04 C01B3/06 C01B3/50 |
| A,D | WO 2017/026914 A1 (PUBLICHNOE AKTSIONERNOE OBSCHESTVO GAZPROM [RU]) 16 February 2017 (2017-02-16) * claims 1-3 * * page 3, paragraph 1st - page 4, last paragraph * | 1-15 | |
| A | A. N. STARTSEV ET AL: "Low Temperature Catalytic Decomposition of Hydrogen Sulfide into Hydrogen and Diatomic Gaseous Sulfur", TOPICS IN CATALYSIS, vol. 56, no. 11, 24 May 2013 (2013-05-24), pages 969-980, XP055552653, NL ISSN: 1022-5528, DOI: 10.1007/s11244-013-0061-y * the whole document * | 1-15 | TECHNICAL FIELDS SEARCHED (IPC) C01B C01C |

The present search report has been drawn up for all claims

| Place of search | Date of completion of the search | Examiner |
|---|---|---|
| The Hague | 19 April 2022 | Gerwann, Jochen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EP 4 180 386 A1

## ANNEX TO THE EUROPEAN SEARCH REPORT
## ON EUROPEAN PATENT APPLICATION NO.

EP 21 31 5243

19-04-2022

| Patent document cited in search report | Publication date | Patent family member(s) | | Publication date |
|---|---|---|---|---|
| WO 2018189637 A1 | 18-10-2018 | NONE | | |
| WO 2017026914 A1 | 16-02-2017 | RU | 2600375 C1 | 20-10-2016 |
| | | WO | 2017026914 A1 | 16-02-2017 |

EPO FORM P0459

For more details about this annex : see Official Journal of the European Patent Office, No. 12/82

REFERENCES CITED IN THE DESCRIPTION

**Patent documents cited in the description**

- US 20050191237 A
- US 20160333277 A
- RU 2216506
- RU 2239594
- WO 2017026914 A
- US 20070292337 A
- US 2017021322 A
- WO 2017118572 A
- WO 2017118573 A
- EP 1080059 A
- US 20100160694 A
- US 8629190 B
- US 2890931 A
- US 4033410 A

**Non-patent literature cited in the description**

- **STARTSEV A. N. et al.** Low-temperature catalytic decomposition of hydrogen sulphide into hydrogen and diatomic gaseous sulphur. *Top. Catal.,* 2013, vol. 56 (11), 969-980
- **ZAGORUIKO A.** Low-temperature chemisorption-enhanced catalytic decomposition of hydrogen sulphide: Thermodynamic analysis and process concept. *Catalysis Today,* 2019, vol. 329, 171-176
- Compendium of Chemical Terminology. 1997
- *Open Journal of Inorganic Non-Metallic Materials,* 2013, vol. 3, 37